(19) Europäisches Patentamt
European Patent Office
Office européen des brevets

(11) EP 2 027 863 A1

(12) EUROPEAN PATENT APPLICATION
published in accordance with Art. 153(4) EPC

(43) Date of publication:
25.02.2009 Bulletin 2009/09

(51) Int Cl.:
A61K 31/702 (2006.01)    A23K 1/16 (2006.01)
A61K 31/7016 (2006.01)   A61P 1/00 (2006.01)
A61P 1/16 (2006.01)      A61P 3/02 (2006.01)
A61P 37/08 (2006.01)     A23L 1/30 (2006.01)

(21) Application number: 07743777.0

(22) Date of filing: 21.05.2007

(86) International application number:
PCT/JP2007/060343

(87) International publication number:
WO 2007/138905 (06.12.2007 Gazette 2007/49)

(84) Designated Contracting States:
AT BE BG CH CY CZ DE DK EE ES FI FR GB GR
HU IE IS IT LI LT LU LV MC MT NL PL PT RO SE
SI SK TR
Designated Extension States:
AL BA HR MK RS

(30) Priority: 30.05.2006 JP 2006149169

(71) Applicant: Ensuiko Sugar Refining Co.Ltd.
Chuo-ku
Tokyo 103-0012 (JP)

(72) Inventors:
• USHIDA, Kazunari
Nishinomiya-shi, Hyogo 662-0098 (JP)

• SAITO, Sanshiro
Chuo-ku,Tokyo 104-0044 (JP)
• SATO, Toshiro
Chuo-ku, Tokyo 104-0044 (JP)
• ISOBE, Yosuke
Chuo-ku, Tokyo 104-0044 (JP)
• FUJITA, Koki
Chuo-ku, Tokyo 103-0012 (JP)
• ITO, Tetsuya
Chuo-ku Tokyo 103-0012 (JP)

(74) Representative: Chapman, Helga Claire
Chapman Molony
20 Staple Gardens
Winchester
SO23 8SR (GB)

(54) INTESTINAL EOSINOPHIL-SUPPRESSING COMPOSITION

(57)    To promote the growth of human beings or animals, prevent the onset of diarrhea and prevent or ameliorate various diseases, it is intended to provide a substance which has an effect of decreasing eosinophils in the intestinal tract and a feed comprising the same as a feed additive. Namely, an intestinal eosionphil-suppressing composition which contains an α-linked galactooligosaccharide as the active ingredient. When added in an amount of from 0.01 to 10% by weight, in terms of the saccharide, to an animal feed, this composition is useful in providing an intestinal eosinophil-suppressing feed.

FIG.1

EP 2 027 863 A1

**Description**

TECHNICAL FIELD

The present invention relates to an intestinal eosinophil-suppressing composition. In particular, the present invention relates to a composition suppressing infiltration of eosinophils into intestinal tract to prevent various diseases related to eosinophils.

BACKGROUND ART

As a result of advancement in breeding technology in terms of nutrition in recent years, excellent feed conversion ratio has been achieved in livestock production. However, the onset of infectious diseases caused by harmful microorganisms remains a difficult and unsolvable problem causing serious damage in livestock production. Contact between livestock and pathogenic microorganisms causes these infectious diseases. Therefore, maintaining a sanitary barn is important.

One factor that degrades sanitation conditions is softening of stool in livestock. Symptoms such as diarrhea and loose stool inhibit growth of livestock, in addition to degrading the sanitation conditions of a barn. Moreover, energy cost of drying feces for disposal or recycling increases. Therefore, softening of stool in livestock has become a significant problem in the livestock industry in recent years.

Conventionally, numerous antibiotics have been used in livestock feed to prevent infectious diseases. However, as concern rises for human infection of antibiotic-resistant microorganisms, a movement to restrict use of antibiotics in feed is spreading worldwide. In other countries that have stopped administration of growth-promoting feed additives, diarrhea and illnesses particularly in weaning-stage piglets are increasing. As a result, therapeutic use of antibiotics is increasing. While risks involving antibiotics cannot be completely eradicated, use of antibiotics is currently unavoidable to maintain growth of livestock.

DISCLOSURE OF INVENTION

Eosinophils are ordinarily not found in the intestines of normal mammals. However, recent studies have shown that eosinophils can be found localized in the small intestines of currently healthy pigs bred in pig farms. While the underlying mechanism of this discovery is unclear, illnesses of which the small intestine is the primary site are increasing in particular in recent years. Therefore, a relationship between the presence of eosinophils in the intestinal tract and the illnesses is suspected. In addition to causing diarrhea and suppressing growth, the presence of eosinophils in the intestinal tract also damages the intestinal tract, causing various illnesses including infection. Various symptoms accompanying diarrhea and loose stool are serious problems that occur not only in livestock, but also humans and pets.

Use of β-linked galactooligosaccharide as a feed additive has been proposed (Japanese Patent Application Laid-open Nos. H05-184308, S62-138147, H05-219897, 2001-103911, and H01-151520). β-linked galactooligosaccharide is considered to have effects such as conditioning intestinal bacterial flora. However, these substances do not reduce eosinophils spontaneously occurring in the intestinal tract.

While a substance that suppresses eosinophils present in the intestinal tract is currently unknown, such a substance is demanded. Therefore, an object of the present invention is to provide a substance that has an effect of reducing eosinophils in the intestinal tract and use of the substance.

After keen research into the above-described issues, the inventors of the present invention have discovered that the issues can be resolved through administration of a composition comprising α-linked galactooligosaccharide as a main ingredient to an animal and the like, thereby arriving at the present invention. In other words, the present invention provides an intestinal eosinophil-suppressing composition in which α-linked galactooligosaccharide is an active ingredient. α-Linked galactooligosaccharide is known to promote the growth of beneficial bifidobacteria and have a caries inhibitory effect (Japanese Patent Application Laid-open No. H05-140178, WO2003/101464, and WO2002/18614). Bifidobacteria produces short-chain fatty acids, such as butyric acid, propionic acid, and acetic acid, in the intestines, thereby reducing the pH level of the intestinal tract and suppressing growth of harmful bacteria, such as *Escherichia coli.* Moreover, the produced short-chain fatty acids stimulate the intestinal tract, thereby prompting bowel movement and ameliorating constipation and diarrhea. Therefore, α-linked galactooligosaccharide was not known to provide a function of suppressing infiltration of eosinophils into the intestinal tract.

α-linked galactooligosaccharide is one or more types of a compound expressed, for example, by the following expression:

$$\alpha-(Gal)_n \qquad (1)$$

(where, Gal is galactose and n is an integer of 2 to 10).

In this case, content of α-linked galactooligosaccharide as α-galactosyl disaccharide is preferably 10% to 100% by weight. α-Linked galactooligosaccharide is preferably prepared by lactose being hydrolyzed, and galactose separated from the hydrolysate being condensation-reacted under the presence of α-galactosidase.

α-Linked galactooligosaccharide can be a mixture of one or more types of the compound expressed by the following expression:

$$\alpha-(Gal)_n \qquad (1)$$

(where, Gal is galactose and n is an integer of 2 to 10), and one or more types of a compound (also referred to, hereafter, as α-galactosyl glucose) expressed by the following expression:

$$\alpha-(Gal)_n Glc \qquad (2)$$

(where, Gal is galactose, Glc is glucose, and n is an integer of 1 to 9).

In this case, content of α-linked galactooligosaccharide as α-galactosyl disaccharide is preferably 10% to 80% by weight. Content of α-linked galactooligosaccharide as α-galactosyl glucose expressed in the expression (2) is preferably 10% to 70% by weight. α-Linked galactooligosaccharide is preferably prepared by lactose being hydrolyzed, and hydrolysate including galactose and glucose being condensation-reacted under the presence of α-galactosidase.

The α-linked galactooligosaccharide composition of the invention is preferably used as at least one type within a group including drugs for intestinal disorders, growth stimulants, drugs for preventing and treating enlargement of edemas and lymphoid follicles in the intestinal tract, and drugs for preventing and treating allergies.

The α-linked galactooligosaccharide composition of the invention can be applied to humans, pets, livestock, and the like. The α-linked galactooligosaccharide composition is preferably applied to livestock, more preferably pigs, and still more preferably piglets.

Therefore, the present invention also provides an intestinal eosinophil-suppressing feed for animals to which an intestinal eosinophil-suppressing composition has been added, the intestinal eosinophil-suppressing composition comprising α-linked galactooligosaccharide as an active ingredient.

In the intestinal eosinophil-suppressing feed for animals, content of α-linked galactooligosaccharide is preferably 0.01% to 10% by weight in terms of the saccharide, α-linked galactooligosaccharide being one or more types of a compound expressed by the following expression:

$$\alpha-(Gal)_n \qquad (1)$$

(where, Gal is galactose and n is an integer of 2 to 10).

In the intestinal eosinophil-suppressing feed for animals, content of α-linked galactooligosaccharide is preferably 0.01% to 10% by weight in terms of the saccharide, α-linked galactooligosaccharide including a mixture of one or more types of the compound expressed by the following expression:

$$\alpha-(Gal)_n \qquad (1)$$

(where, Gal is galactose and n is an integer of 2 to 10), and one or more types of a compound expressed by the following expression:

$$\alpha-(Gal)_n Glc \qquad (2)$$

(where, Gal is galactose, Glc is glucose, and n is an integer of 1 to 9).

The inventors have also discovered that α-linked galactooligosaccharide has an effect of conditioning liver function. Therefore, the present invention also provides a liver-function enhancing composition in which an active ingredient is α-linked galactooligosaccharide.

When the α-linked galactooligosaccharide composition of the invention is, for example, added to feed and administered to animals, infiltration of eosinophils into the intestinal tract of animals can be suppressed. As a result, growth of animals can be promoted, in addition to treating and preventing diarrhea, edemas, and infections. These effects are particularly advantageous in piglets that are susceptible to diarrhea. Moreover, the α-linked galactooligosaccharide composition of the invention conditions liver function, thereby suppressing diseases related to liver function.

BRIEF DESCRIPTION OF DRAWINGS

[Fig. 1] Diagram of calculated values of eosinophils in a center portion of the ileum when piglets are fed feeds respectively including intestinal eosinophil-suppressing compositions of the present invention and a comparative composition and dissected.

[Fig. 2] Diagram of calculated values of GOT when piglets are fed feeds including intestinal eosinophil-suppressing compositions of the present invention and a comparative composition.

BEST MODE(S) FOR CARRYING OUT THE INVENTION

α-Linked galactooligosaccharide that is an active ingredient of an intestinal eosinophil-suppressing composition of the invention refers to oligosaccharide having an α-galactosyl group. α-Linked galactooligosaccharide is preferably one or more types of a compound expressed by the following expression:

$$\alpha\text{-(Gal)}_n \qquad (1)$$

(where, Gal is galactose and n is an integer of 2 to 10, preferably an integer of 2 to 8).

The compound in the expression (1) is obtained by being extracted from a natural raw material. Alternatively, the compound is obtained by a reaction through which galactose is condensed under the presence of α-galactosidase. In the latter case, α-linked galactooligosaccharide that includes a plurality of oligosaccharides, such as α-galactosyl disaccharide, α-galactosyl trisaccharide, α-galactosyl tetrasaccharide, or higher, is ordinarily obtained. Galactose binding sites, number of bonds, and a ratio of these oligosaccharides differ based on the origin of the enzyme being used and reaction type.

Content of α-linked galactooligosaccharide as α-galactosyl disaccharide is preferably 30% to 100% by weight, and more preferably 40% to 100% by weight. When the content of α-linked galactooligosaccharide as α-galactosyl disaccharide is less than 30% per weight, intake of an effective amount may become difficult to achieve.

α-Linked galactooligosaccharide may be a mixture of the compound in the expression (1) and one or more types of a compound expressed by the following expression:

$$\alpha\text{-(Gal)}_n\text{Glc} \qquad (2)$$

(where, Gal is galactose, Glc is glucose, and n is an integer of 1 to 9, preferably an integer of 1 to 7).

The compound in the expression (2) is obtained by being extracted from a natural raw material. Alternatively, the compound is obtained by a reaction through which galactose and glucose are condensed under the presence of α-galactosidase. Through condensation reaction, α-linked galactooligosaccharide including both the oligosaccharide expressed by the expression (1) and the α-galactosyl glucose expressed by the expression (2) is ordinarily obtained. Galactose binding sites, number of bonds, and a ratio of these oligosaccharides differ based on composition of galactose and glucose in the raw material, the origin of the enzyme being used, and reaction type.

In the above-described mixture, content of α-linked galactooligosaccharide as α-galactosyl disaccharide is preferably 10% to 80% by weight. Content of α-linked galactooligosaccharide as α-galactosyl glucose is preferably 10% to 70% by weight. When the content of α-linked galactooligosaccharide as α-galactosyl glucose is less than 10% by weight, effect may not be sufficiently achieved.

The galactose serving as a material for condensation reaction can be prepared by hydrolysis being performed on α-galactosyl or β-galactosyl oligosaccharides, glycosides, and polysaccharides using enzymes, such as β-galactosidase, α-galactosidase, and β-galactanase, or inorganic acids, such as hydrochloric acid, nitric acid, and phosphoric acid. The α-galactosyl or β-galactosyl oligosaccharides are, for example, melibiose, manninotriose, raffinose, stachyose, planteose, verbascose, galactan, galactomannan, arabinogalactan, rhamnogalactan, galactolipid, ferulic galactose, galactopinitol, galactosylglycerol, galactinol, lactose, lactitol, lactulose, and galactooligosaccharides. Commercially available galactose can also be used.

Preferably, a hydrolysate (mixture of galactose and glucose) is obtained by inexpensive lactose interacting with β-galactosidase or the above-described acid. Galactose is separated from the hydrolysate. Alternatively, the above-described condensation reaction is performed on the mixture as is. Hydrolysis of lactose is performed, for example, under following conditions: commercially-available lactose concentration of 10%(w/w), reaction temperature of 50°C, and β-galactosidase enzyme concentration of 20U/g-lactose.

Galactose used in dehydrocondensation reaction to synthesize only the α-linked galactooligosaccharide in the expression (1) is separated from the hydrolysate of lactose by use of ion exchange chromatography, activated carbon column, and the like. Active carbon of 1% solid content is added to a galactose fraction. The galactose fraction is then heated for an hour at 95°C. A clarified liquid is obtained using a pressure filter. The clarified liquid is desalted, and then concentrated until a concentration of 54%(w/w) is reached in a vacuum crystallizer at 55°C. Galactose seed crystals are added to the clarified liquid, and the clarified liquid is held at 55°C for three hours. The clarified liquid is then cooled at a rate of 1°C per hour to 40°C, and the galactose is crystallized. Crystals are collected by centrifugal separation. High-purity galactose is ultimately obtained.

Yeast of 2% solid content can be added to the hydrolyzed liquid obtained above. The hydrolyzed liquid is then held at 33°C for 93 hours. As a result, the yeast is assimilate glucose, thereby increasing purity of galactose. Further crystallization is performed, allowing high-purity galactose to be obtained.

Origin of the enzyme, α-galactosidase, used in condensation reaction is not particularly limited, as long as dehydrocondensation reaction occurs with galactose or galactose and glucose as the materials and α-linked galactooligosaccharide can be synthesized. Specific examples of α-galactosidase are: filamentous fungi, such as *Aspergillus niger, Aspergillus oryzae, Aspergillus pulverulentus, Penicillium purpurogenum, Penicillium citrinum, Penicillium multicolor, Trichoderma viride, Mortierella vinacea,* S. *carlsbergensis,* and *Candida guilliermondii;* basidiomycete, such as *Pycnoporus cinnabarinus;* bacteria, such as *Bacillus megaterium, Streptococcus bovis, Pseudomonas fluorescens,* and *Diplococcus pneumoniae;* yeast, such as *Saccharomyces cervisiae; Vicia sativa;* coffee beans; and α-galactosidase produced by humans. α-galactosidase produced by *Aaspergillus niger* is preferable in terms of yield and the like. α-galactosidase originating from *Aspergillus niger* (APC-9319 strain [FERM BP-7680], Sumizyme AGS-L, and the like) is more preferable.

Condensation reaction conditions, such as concentrations of galactose and appropriate glucose used in the condensation reaction, amount of the enzyme α-galactosidase added, pH level, and reaction temperature, are adjusted accordingly. The concentration of galactose is ordinarily 5% to 90% by weight, preferably 40% to 70% by weight. The concentration of the enzyme is ordinarily 1 to 100U/g galactose, and preferably 5 to 30U/g galactose. The pH level is ordinarily in a range of 3.0 to 10.0, preferably 4.0 to 8.0. The reaction temperature is ordinarily 20°C to 90°C, preferably 40°C to 70°C. Reaction time differs based on an amount of enzyme used, but is ordinarily 1 to 240 hours, preferably 24 to 90 hours.

To produce a higher concentration of galactose and efficiently produce α-linked galactooligosaccharide treating galactose with an α-galactosidase to effect a preliminary dehydrocondensation reaction as a prereaction followed by concentrating the reaction solution under vacuum, and prior to the dehydrocondensation reaction as a principal reaction. The concentrated liquid can be removed, and the principal reaction can be performed in a separate series from the prereaction. Alternatively, the principal reaction can be performed in a same series as the prereaction without the concentrated liquid being removed.

The solution after condensation reaction includes unreacted galactose in addition to α-linked galactooligosaccharide. The reactants can be used as is. Alternatively, the reactants can be fractionated by methods such as ion exchange chromatography, activated carbon column chromatography, gel-filtration column chromatography, utilization by a certain microorganism, and solid-liquid separation using differences in solubility.

When α-linked galactooligosaccharide including only α-$(Gal)_n$ (n is 2 to 10) is prepared using lactose as the material, procedures are required to be performed, such as separating galactose from the lactose hydrolysate, and removing unreacted galactose from the condensation reaction product. The α-linked galactooligosaccharides expressed in the expression (1) and the expression (2) can be synthesized by the lactose hydrolysate being directly interacted with α-galactosidase, without the fraction operation being performed after lactose hydrolysis. This method is suitable for use in the feed industry where economic efficiency is required, because the above-described purification process leading to increased cost can be omitted and the lactose hydrolysate can be efficiently used. Moreover, α-linked galactooligosaccharide including α-galactosyl glucose is also preferable in terms of having an effect of significantly promoting growth of piglets with a small amount of intake.

The intestinal eosinophil-suppressing composition of the invention, manufactured as described above, is in a syrup-like liquid form and can be used as is. Moreover, the intestinal eosinophil-suppressing composition can be used in combination with sitologically and pharmacologically acceptable excipients (lactose, sucrose, glucose, cornstarch, gelatin, starch, dextrin, silicic acid anhydride, calcium phosphate, calcium dihydrogen, aluminum silicate, magnesium oxide, aluminum hydroxide, magnesium stearate, calcium stearate, sodium bicarbonate, yeast, water, sorbitol, ethanol, propylene glycol, glycerin, ethylene glycol, polyethylene glycol, fatty oil, and the like), binders, disintegrating agents, stabilizers, lubricants, demulcents, preservatives, antifungal agents, flavoring agents, sweetening agents, and the like.

The liquid or solid intestinal eosinophil-suppressing composition of the invention is processed accordingly into powder, granules, tablets, biscuits, flakes, soft capsules, hard capsules, and syrups.

The intestinal eosinophil-suppressing composition of the invention is mainly provided as functional foods and health foods in the above-described forms, or intestinal eosinophil-suppressing food and intestinal eosinophil-suppressing feed for animals including the composition in the above-described forms.

Specific examples of the above-described foods are: food products, such as bread, buckwheat noodles, Japanese wheat noodles, fried noodles, rice crackers, cookies, biscuits, candy, and soup; dairy products, such as milk, yogurt, and ice cream; and drinks, such as carbonated beverages, soft drinks, fruit juices, and medicinal drinks.

The intestinal eosinophil-suppressing composition of the invention is preferably combined with animal feed. The content of $\alpha$-linked galactooligosaccharide is ordinarily 0.01% to 10% by weight, preferably 0.01% to 5% by weight, more preferably 0.025% to 2.5% by weight in terms of the saccharide, in that the intestinal eosinophil-suppressing function, and intestinal conditioning effect, growth-promoting effect, and the like based on the intestinal eosinophil-suppressing function are maximized, $\alpha$-linked galactooligosaccharide being one or more types of a compound expressed by the following expression:

$$\alpha\text{-}(Gal)_n \qquad\qquad (1)$$

(where, Gal is galactose, and n is an integer of 2 to 10, preferably an integer of 2 to 8). When the content of $\alpha$-linked galactooligosaccharide of the expression (1) is less than 0.01% by weight, intake of an effective amount may become difficult to reach. When the content exceeds 10% by weight, excessive ingestion may occur.

More preferably, in terms of preparation at a low manufacturing cost, in the animal feed, the content of $\alpha$-linked galactooligosaccharide is ordinarily 0.01% to 10% by weight, preferably 0.01% to 5% by weight, and more preferably 0.025% to 2.5% by weight in terms of the saccharide, in that the intestinal eosinophil-suppressing function, and intestinal conditioning effect, growth-promoting effect, and the like based on the intestinal eosinophil-suppressing function are maximized, $\alpha$-linked galactooligosaccharide being a mixture of one or more types of a compound expressed by the following expression:

$$\alpha\text{-}(Gal)_n \qquad\qquad (1)$$

(where, Gal is galactose, and n is an integer of 2 to 10, preferably an integer of 2 to 8), and one or more types of a compound expressed by the following expression:

$$\alpha\text{-}(Gal)_n Glc \qquad\qquad (2)$$

(where, Gal is galactose, Glc is glucose, and n is an integer of 1 to 9, preferably an integer of 1 to 7) . When the content of $\alpha$-linked galactooligosaccharide of the expression (1) is less than 0.01% by weight, intake of an effective amount may become difficult to reach. When the content exceeds 10% by weight, excessive ingestion may occur.

Ordinarily used feed materials can be used as the feed to which the intestinal eosinophil-suppressing composition of the invention is added. The feed materials are, for example: grains, such as rice, brown rice, rye, wheat, barley, corn, corn gluten meal, corn germ meal, white fish meal, milo; offals, such as wheat bran, defatted rice bran, and corn gluten meal; oils and fats, such as soybean meal, rapeseed oil and fat, soybean oil and fat, powdered refined tallow, and animal oil and fat; inorganic salt, such as magnesium sulfate, iron sulfate, copper sulfate, zinc sulfate, potassium iodide, cobalt sulfate, calcium carbonate, tricalcium phosphate, sodium chloride, calcium phosphate, and choline chloride; amino acids, such as lysine, and methionine; vitamins, such as vitamin A, vitamin B1, vitamin B2, vitamin B6, vitamin B12, vitamin D, vitamin E. calcium pantothenate, nicotinamide, and folic acid; synthetic milk, such as powered skim milk; alfalfa meal; fish meal; and dried whey.

The composition of the invention can be appropriately diluted with water and the like, and injected into the blood. As long as the intestinal eosinophil-suppressing effect of the $\alpha$-linked galactooligosaccharide is not lost, the intestinal eosinophil-suppressing composition of the invention can be provided in combination with other drugs.

Intake of the intestinal eosinophil-suppressing composition of the invention in which the active ingredient is the α-linked galactooligosaccharide is adjusted accordingly based on the intended purpose, such as use in drugs for intestinal disorders, growth stimulants, drugs for preventing and treating enlargement of edema and lymphoid follicles in the intestinal tract, drugs for preventing and treating allergies, and liver-function enhancers, the age of the human or animal, the weight of the human or animal, the administration method, and the like. Daily intake of α-linked galactooligosaccharide by humans and animals is ordinarily 1mg to 20g, preferably 10mg to 5g, more preferably 10mg to 4g.

Hereafter, the invention will be described in further detail using examples. However, the invention is not limited to the examples.

[Examples 1 and 2]

(Preparation of α-linked galactooligosaccharide)

An oligosaccharide composition (referred to, hereafter as α-GOS A) including only the α-linked galactooligosaccharide in the expression (1) as the α-linked galactooligosaccharide and an α-linked galactooligosaccharide (referred to, hereafter as α-GOS B) including the α-linked galactooligosaccharide in the expression (1) and the α-galactosyl glucose in the expression (2) were prepared by the following procedures.

(Preparation of α-GOS A)

Lactose (400kg) was dissolved in water and adjusted to a concentration of 10%(w/w) and a pH level of 6.5. β-galactosidase (product name: Lactoless L3, DAIWA KASEI K. K. ) of 20U/g-lactose was added to the lactose solution. The lactose solution was then hydrolysis-reacted at 50°C. Yeast of 2% solid content was then added. The hydrolysate was held at 33°C for 93 hours.

After being reacted, activated carbon of 1% solid content was added. The hydrolysate was heated for an hour at 95°C. A clarified liquid was obtained using a pressure filter. A solution with a galactose purity of 77.9% was prepared. The solution was crystallized by a cooling crystallization method, and separated in to molasses and crystals by a centrifuge. The crystals were then washed with cold water, allowing 65kg of galactose crystals with a purity of 97.0% to be obtained at a yield of 37%.

The galactose crystals were completely dissolved at 75°C and cooled to 65°C. The galactose concentration was then adjusted to 70%(w/w). α-Galactosidase derived from *Aaspergillus niger* strain APC-9319 was added, and condensation reaction was induced. After three hours of reaction, the reaction liquid was depressurized while being held at 65°C, and concentrated to 90%(w/w). The α-galactosidase derived from *Aspergillus niger* strain APC-9319 was further added, and the reaction liquid was reacted for 40 hours at 65°C. The reaction liquid was heated for an hour at 95°C and enzymatic reaction was stopped. In addition, activated carbon of 2% solid content was added, and the reaction liquid was decolorized. Subsequently, a clarified liquid was obtained by pressure-filtering. The clarified liquid was desalted, and then concentrated to 50%(w/w) using an evaporator, forming a fractionation solution. α-Linked galactooligosaccharide fractions were collected at a yield of 90% or more by a simulated moving bed process. The obtained α-linked galactooligosaccharide fractions were concentrated using the evaporator, desalted, and then spray-dried.

(Preparation of α-GOS B)

Thirty kilograms of lactose was dissolved to Bx.8 solid content and adjusted to a pH level of 6.5. Then, 203mL (663,810U) of β-galactosidase (product name: Lactoless L3, DAIWA KASEI K. K.) was added to the lactose solution. The lactose solution was then hydrolyzed for 94 hours at 50°C. After reaction, 500g of activated carbon (Shirasagi A, manufactured by Japan EnviroChemicals, Ltd.) was added, and the reaction liquid was heated for an hour at 95°C. Then, 3kg of diatomaceous earth (Radiolite #100, manufactured by Showa Chemical Industry Co., Ltd) was added to the reaction liquid. The reaction liquid was filtered by a pressure filter. Undecomposed lactose within the reaction liquid was 1% or less.

The obtained lactose hydrolyzed liquid was concentrated to Bx.72 solid content by an evaporator, among which 20.8kg solid content was used for condensation reaction. The concentrated lactose hydrolyzed liquid was adjusted to a pH level of 4.5 using hydrochloric acid. Then, 67.3mL (2,019,000U) of α-galactosidase (product name: Sumizyme AGS-L, Shin-nihon-kagaku-kogyo) derived from *Aspergillus niger* was added to the lactose hydrolyzed liquid. The lactose hydrolyzed liquid was heated for 49 hours at 65°C, and condensation reaction was induced. After reaction, the reaction liquid was heated for an hour at 100°C. Five-hundred grams of activated carbon (Shirasagi A) was added to the reaction liquid, and the reaction liquid was filtered using diatomaceous earth (Radiolite #100). Then, after the filtered liquid was the passed through a desalting resin (IRA-404, 3. 6L, IR-200C, 1.8L, manufactured by Organo Corporation), the filtered liquid was further concentrated to Bx.72.

Compositions (unit: % by weight) of the α-GOS A and the α-GOS B obtained above are shown in Table 1.

[Table 1]

| Composition | α-GOS A | α-GOS B |
|---|---|---|
| α-galactosyl disaccharide disaccharide | 57.8 | 10.8 |
| α-galactosyl trisaccharide | 28.6 | - |
| α-galactosyl tetrasaccharide or higher | 13.6 | - |
| α-galactosyl glucose | - | 13.5 |
| Residue | - | 75.7 |
| Total | 100 | 100 |

(Preparation of feed including α-linked galactooligosaccharide)

Feeds (Examples 1 and 2) including the α-linked galactooligosaccharide was prepared with mixture ratios shown in Table 2, by the two types of oligosaccharides described above being added to early-stage artificial milk feed for piglets (product name: SDS No. 1, Nippon Formula Feed Manufacturing Co. , Ltd.). In contrast, a feed mixture using sucrose (product name: granulated sugar, Pearl Ace Corporation) in place of α-linked galactooligosaccharide was also prepared.

[Table 2]

| Ingredient | Comparative Example 1 | Example 1 | Example 2 |
|---|---|---|---|
| Sucrose | 0.25 | - | - |
| α-GOS A | - | 2.6 | - |
| α-GOS B | - | - | 0.25 |
| SDS No. 1 | 99.75 | 97.4 | 99.75 |
| Total (parts by weight) | 100 | 100 | 100 |

(Preparation of test animals)

Nine 21-day-old, cross-bred piglets of suckling age were obtained from Kyoto Animal Inspection Center. The piglets were weighed at introduction and divided into three groups of three piglets such that weight is evenly distributed.

(Administration of feed)

The piglets were housed in groups in piglet pens having a concrete floor covered with sawdust. Temperature of the piglet pens was managed using an electric brooder. The piglets were continuously fed feed to which each test substance is added for 10 consecutive days from introduction. During the test period, the piglets had free access to water. Clinical abnormalities caused by administration of each test substance were not found during the test period. No side effects were observed.

During the 10-day administration period, fecal properties were observed daily and scored in accordance to the following criteria: normal stool 0; loose stool 1; mud-like stool 2; and watery diarrhea 3. A total score was determined for each group on day 10 (Table 3).

Weight and amount of ingestion were measured on day 0, day 3, and day 10 after the test was started. From the measured weight and ingested amount, weight increase and feed demand rate were determined (Table 3).

After testing, the weaning-stage piglets were dissected. Histopathologic tests, measurement of organic acid and immunoglobulin concentrations in intestinal contents, cytotoxic activity and monocyte latex-bead phagocytic capacity of natural killer cells in the blood, blood tests, biochemical examinations, and the like were performed. The piglets were dissected such that difference in dissection time among groups is kept to a minimum, in a following manner, for example: one piglet from contrast group → one piglet from α-GOS A-administered group → one piglet from α-GOS B-administered group → one piglet from contrast group. Each piglet was intramuscularly injected with Ketalar and Stressnil, and exsanguinated. During exsanguination, blood was collected from the abdominal aorta. Other than the following items, no significant differences between each example were found in the histopathologic tests, the measurement of organic acid and immunoglobulin concentrations in intestinal contents, the cytotoxic activity and monocyte latex-bead phagocytic capacity of natural killer cells in the blood, the blood tests, or the biochemical examinations.

(Effect of suppressing infiltration of intestinal tract by eosinophils)

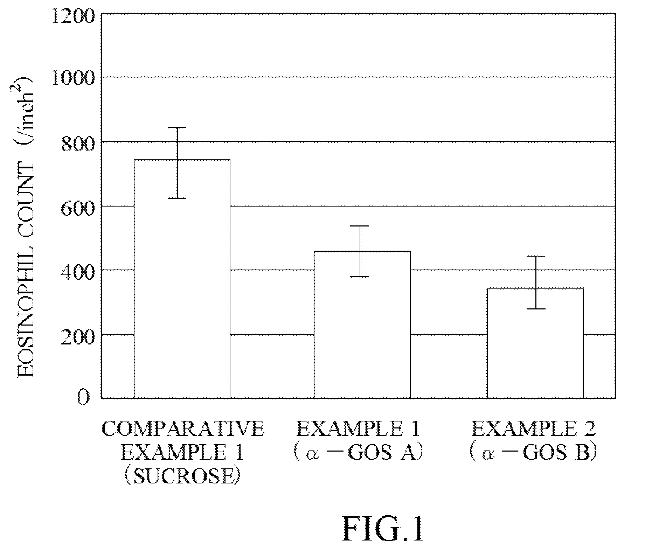

The number of eosinophils in a small intestine sample were counted using Luna stain (Fig. 1 and Table 3). A significant difference was found in the average number of eosinophils in the center portion of the ileum, the average number being 765.4/inch$^2$ in the comparative example 1, 468.3/inch$^2$ in the example 1, and 363.9/inch$^2$ in the example 2. The numbers of eosinophils in the center portion of the ileum in the example 1 ($\alpha$-GOS A) and the example 2 ($\alpha$-GOS B) are significantly lower compared to that of the comparative example 1 as control group. Therefore, it is clear that the infiltration of the small intestine by the eosinophils is suppressed by administration of $\alpha$-GOS A and $\alpha$-GOS B. Because weaning-stage piglets are susceptible to pathogenic diarrhea as a result of stress from being forcibly separated from their mothers, stimulation of the immune system of the digestive tract is particularly important. Eosinophils are frequently found in allergic states, such as asthma. It is also known that secretory granules from eosinophils cause tissue damage. In terms of Th1/Th2 balance as well, infiltration by large amounts of Th2-type eosinophils is undesirable. To prevent pathogenic diarrhea, it is preferable that the Th1-type immune system is enhanced in the digestive tract. Although this does not limit the intentions the invention, suppression of infiltration by eosinophils through administration of $\alpha$-GOS A and $\alpha$-GOS B can be considered to indicate enhancement of the Th1-type immune system.

(Effects of improving intestinal disorders and promoting growth)

In the example 1 in which the piglets were fed $\alpha$-GOS A, improvements in fecal properties, weight increase, and feed demand rate could be seen (Table 3). In the example 2 in which the piglets were fed $\alpha$-GOS B, further improvements in fecal properties, weight increase, and feed demand rate could be seen (Table 3). Highest importance is placed on weight increase in terms of pig farm operation.

(Effect of improving liver function)

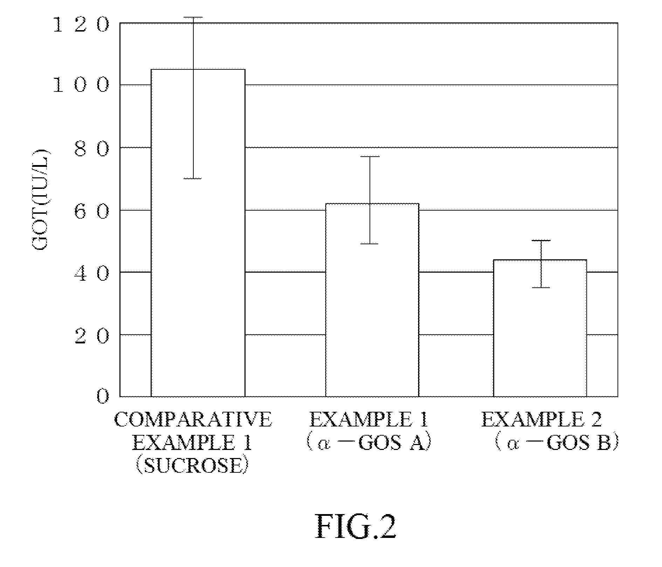

Whole blood and blood serum were separated from the above-described collected blood, and glutamic oxaloacetic transaminase (GOT) was measured (Fig. 2 and Table 3). A significant difference was found in the average GOT concentration, the average GOT concentration being 104.3IU/L in the comparative example 1, 62.0IU/L in the example 1, and 43.3IU/L in the example 2. Because the GOT concentration in the blood significantly decreased as a result of administration of $\alpha$-GOS A and $\alpha$-GOS B, the possibility of improvement in liver functions is suggested.
[Table 3]

| Content of feed mixture | | Comparative Example 1 | Example 1 | Example 2 |
|---|---|---|---|---|
| | | Sucrose-a dded | $\alpha$-GOS A-added | $\alpha$-GOS B-added |
| Weight (kg) | Day 0 | 4.7 | 4.6 | 5.0 |
| | Day 3 | 4.7 | 4.9 | 4.8 |
| | Day 10 | 5.1 | 5.3 | 6.1 |
| Weight increase (kg) | Day 0 to Day 10 | 0.4 | 0.7 | 1.1 |
| Feed consumption (kg/animal) | Day 0 to Day 3 | 0.13 | 0.20 | 0.07 |
| | Day 3 to Day 10 | 0.87 | 1.20 | 1.33 |
| | Day 0 to Day 10 | 1.00 | 1.40 | 1.40 |
| Feed demand rate | Day 0 to Day 10 | 2.31 | 1.91 | 1.27 |

(continued)

| Content of feed mixture | | | Comparative Example 1 | Example 1 | Example 2 |
|---|---|---|---|---|---|
| | | | Sucrose-a dded | α-GOS A-added | α-GOS B-added |
| Fecal property (scored value) | | Day 0 | 0 | 0 | 0 |
| | | Day 1 | 0 | 0 | 0.7 |
| | | Day 2 | 0 | 0 | 0.7 |
| | | Day 3 | 0.7 | 0 | 0 |
| | | Day 4 | 0.3 | 0 | 0 |
| | | Day 5 | 0.7 | 0.3 | 0 |
| | | Day 6 | 0.7 | 0.3 | 0 |
| | | Day 7 | 0 | 0 | 0 |
| | | Day 8 | 0.3 | 0.3 | 0 |
| | | Day 9 | 0.7 | 0.7 | 0 |
| | | Day 10 | 0.7 | 1.7 | 0 |
| | | Total score | 4.0 | 3.3 | 1.3 |
| Eosinophils in center portion of ileum (/inch$^2$) | | | 765.4 | 468.3 | 363.9 |
| GOT (IU/L) | | | 104.3 | 62.0 | 43.3 |

The intestinal eosinophil-suppressing composition of the invention is effective as a drug for intestinal disorders for humans and animals based on this function. Because healing from diarrhea and the like leads to increased appetite, the intestinal eosinophil-suppressing composition of the invention is also effective as a growth stimulant. The intestinal eosinophil-suppressing composition is particularly effective as a drug for intestinal disorders and a growth stimulant for piglets, which are susceptible to diarrhea.

The intestinal eosinophil-suppressing composition of the invention is also suitable as a drug for preventing and treating allergies, and a drug for preventing and treating enlargement of edema and lymphoid follicles.

The intestinal eosinophil-suppressing composition of the invention can also be used as a liver-function enhancer.

**Claims**

1. An intestinal eosinophil-suppressing composition comprising α-linked galactooligosaccharide as an active ingredient.

2. The intestinal eosinophil-suppressing composition according to claim 1, wherein the α-linked galactooligosaccharide is one or more types of a compound expressed by the following expression:

$$\alpha-(Gal)_n \qquad (1)$$

(where, Gal is galactose and n is an integer of 2 to 10).

3. The intestinal eosinophil-suppressing composition according to claim 2, wherein content of α-linked galactooligosaccharide as α-galactosyl disaccharide is 10% to 100% by weight of all oligosaccharides.

4. The intestinal eosinophil-suppressing composition according to claim 2 or 3, wherein α-linked galactooligosaccharide is prepared by lactose being hydrolyzed, and galactose separated from a hydrolysate being condensation-reacted under presence of α-galactosidase.

5. The intestinal eosinophil-suppressing composition according to claim 1, wherein α-linked galactooligosaccharide is a mixture of one or more types of a compound expressed by the following expression:

$$\alpha-(Gal)_n \qquad (1)$$

(where, Gal is galactose and n is an integer of 2 to 10), and one or more types of a compound expressed by the following expression:

$$\alpha-(Gal)_n Glc \qquad (2)$$

(where, Gal is galactose, Glc is glucose, and n is an integer of 1 to 9).

6.  The intestinal eosinophil-suppressing composition according to claim 5, wherein content of $\alpha$-linked galactooligosaccharide as $\alpha$-galactosyl disaccharide is 10% to 80% by weight, and content of $\alpha$-linked galactooligosaccharide as $\alpha$-galactosyl glucose in the expression (2) is 10% to 70% by weight.

7.  The intestinal eosinophil-suppressing composition according to claim 5 or 6, wherein $\alpha$-linked galactooligosaccharide is prepared by lactose being hydrolyzed, and hydrolysate including galactose and glucose being condensation-reacted under presence of $\alpha$-galactosidase.

8.  The intestinal eosinophil-suppressing composition according to any one of claims 1 to 7, wherein the intestinal eosinophil-suppressing composition is used as at least one type within a group including drugs for intestinal disorders, growth stimulants, drugs for preventing and treating enlargement of edemas and lymphoid follicles in intestinal tract, and drugs for preventing and treating allergies.

9.  The intestinal eosinophil-suppressing composition according to claim 8, wherein the intestinal eosinophil-suppressing composition is for animals.

10. An intestinal eosinophil-suppressing feed for animals to which an intestinal eosinophil-suppressing composition according to claim 9 is added.

11. The intestinal eosinophil-suppressing feed for animals according to claim 10, wherein content of $\alpha$-linked galactooligosaccharide is 0.01% to 10% by weight in terms of the saccharide, $\alpha$-linked galactooligosaccharide being one or more types of a compound expressed by the following expression:

$$\alpha-(Gal)_n \qquad (1)$$

(where, Gal is galactose and n is an integer of 2 to 10).

12. The intestinal eosinophil-suppressing feed for animals according to claim 10, wherein content of $\alpha$-linked galactooligosaccharide is 0.01% to 10% by weight in terms of the saccharide, $\alpha$-linked galactooligosaccharide including a mixture of one or more types of a compound expressed by the following expression:

$$\alpha-(Gal)_n \qquad (1)$$

(where, Gal is galactose and n is an integer of 2 to 10), and one or more types of a compound expressed by the following expression:

$$\alpha-(Gal)_n Glc \qquad (2)$$

(where, Gal is galactose, Glc is glucose, and n is an integer of 1 to 9).

**13.** A liver-function enhancing composition comprising α-linked galactooligosaccharide as an active ingredient.

FIG.1

FIG.2

<div align="center">**INTERNATIONAL SEARCH REPORT**</div>

| | International application No. |
|---|---|
| | PCT/JP2007/060343 |

**A. CLASSIFICATION OF SUBJECT MATTER**
*A61K31/702*(2006.01)i, *A23K1/16*(2006.01)i, *A61K31/7016*(2006.01)i, *A61P1/00*(2006.01)i, *A61P1/16*(2006.01)i, *A61P3/02*(2006.01)i, *A61P37/08*(2006.01)i, *A23L1/30*(2006.01)n

According to International Patent Classification (IPC) or to both national classification and IPC

**B. FIELDS SEARCHED**

Minimum documentation searched (classification system followed by classification symbols)
A61K31/702, A23K1/16, A61K31/7016, A61P1/00, A61P1/16, A61P3/02, A61P37/08, A23L1/30

Documentation searched other than minimum documentation to the extent that such documents are included in the fields searched
Jitsuyo Shinan Koho          1922-1996    Jitsuyo Shinan Toroku Koho   1996-2007
Kokai Jitsuyo Shinan Koho    1971-2007    Toroku Jitsuyo Shinan Koho   1994-2007

Electronic data base consulted during the international search (name of data base and, where practicable, search terms used)
CAPLUS(STN), MEDLINE(STN), BIOSIS(STN), EMBASE(STN)

**C. DOCUMENTS CONSIDERED TO BE RELEVANT**

| Category* | Citation of document, with indication, where appropriate, of the relevant passages | Relevant to claim No. |
|---|---|---|
| X<br>A | Hiroyuki HASHIMOTO et al., "α-Galactosyl Ki o Fukumu Oligosaccharide To no Gosei to Sono Kino", JOURNAL OF APPLIED GLYSCOSCIENCE , 2004, Vol.51, No.2, pages 169 to 176 | 1-12<br>13 |
| X<br>A | SONOYAMA, K. et al., Allergic Airway Eosinophilia Is Suppressed in Ovalbumin-Sensitized Brown Norway Rats Fed Raffinose and α-Linked Galactooligosaccharide, Journal of Nutrition, 2005, Vol.135, No.3, pp.538-543 | 1-12<br>13 |
| X<br>A | WO 02/18614 A1  (Amano Enzyme Inc.), 07 March, 2002 (07.03.02), Full text & EP 1321527 A1        & US 2004/101938 A1 | 1-12<br>13 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>06 June, 2007 (06.06.07) | Date of mailing of the international search report<br>19 June, 2007 (19.06.07) |
|---|---|
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

| International application No. |
| --- |
| PCT/JP2007/060343 |

C (Continuation).    DOCUMENTS CONSIDERED TO BE RELEVANT

| Category* | Citation of document, with indication, where appropriate, of the relevant passages | Relevant to claim No. |
| --- | --- | --- |
| X<br>A | WO 03/101464 A1  (Amano Enzyme Inc.),<br>11 December, 2003 (11.12.03),<br>Full text<br>& EP 1514551 A1          & US 2006/234980 A1 | 1-12<br>13 |
| X<br>A | JP 5-140178 A  (Honen Corp.),<br>08 June, 1993 (08.06.93),<br>Full text<br>(Family: none) | 1-12<br>13 |
| X<br>A | JP 5-146273 A  (Honen Corp.),<br>15 June, 1993 (15.06.93),<br>Full text<br>(Family: none) | 1-12<br>13 |
| A | Masao ITO et al., Igaku Shoin Igaku Daijiten,<br>page 2070, 'Lactobacillus Bifidus', issued on<br>01 March, 2003 (01.03.03) | 1-12 |

Form PCT/ISA/210 (continuation of second sheet) (April 2005)

## INTERNATIONAL SEARCH REPORT

| | International application No. |
|---|---|
| | PCT/JP2007/060343 |

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:
The present application has the following two invention groups.
  1. A group of inventions relating to an intestinal eosionphil-suppressing composition which contains an α-linked galactooligosaccharide as the active ingredient as claimed in claims 1 to 12.
  2. A group of invention relating to a liver function-ameliorating composition which contains an α-linked galactooligosaccharide as the active ingredient as claimed in claim 13.
  Although the two invention groups as described above are common to each other in being a remedy which contains an α-linked galactooligosaccharide as the active ingredient, such a remedy had (continued to extra sheet)

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

EP 2 027 863 A1

**INTERNATIONAL SEARCH REPORT**

International application No.

PCT/JP2007/060343

Continuation of Box No.III of continuation of first sheet(2)

been publicly known before the present application, as disclosed by, for example, WO 03/101464 A1, WO 02/18614 A1, etc. Such being the case, there is no special technical feature exceeding prior art that is common to these two invention groups and, therefore, these invention groups are not considered as being so linked as to form a single general inventive concept.

Form PCT/ISA/210 (extra sheet) (April 2005)

18

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H05184308 B
- JP S62138147 B
- JP H05219897 B
- JP 2001103911 A
- JP H01151520 B
- JP H05140178 B
- WO 2003101464 A
- WO 200218614 A